## Europäisches Patentamt

## European Patent Office

## Office européen des brevets

(19)

(11) Numéro de publication : **0 004 510**
**B1**

(12)

# FASCICULE DE BREVET EUROPÉEN

(45) Date de publication du fascicule du brevet :
08.02.84

(51) Int. Cl.³ : **A 61 B 5/04**, G 01 N 27/30,
**A 61 B 5/14**

(21) Numéro de dépôt : 79400181.8

(22) Date de dépôt : 20.03.79

(54) **Tête de mesure permettant l'obtention de signaux de mesures physiologiques, destinée à être mise en place sur ou dans des parties corporelles.**

(30) Priorité : 23.03.78 FR 7808428

(43) Date de publication de la demande :
03.10.79 **Bulletin 79/20**

(45) Mention de la délivrance du brevet :
08.02.84 **Bulletin 84/06**

(84) Etats contractants désignés :
**CH DE FR GB IT SE**

(56) Documents cités :
FR-A- 2 118 657
FR-A- 2 120 787
FR-A- 2 198 638
FR-A- 2 247 725
US-E- 28 990

(73) Titulaire : **ANVAR Agence Nationale de Valorisation de la Recherche**
**13, rue Madeleine Michelis**
**F-92522 Neuilly-sur-Seine (FR)**

(72) Inventeur : **Bernard, Claude**
**5, square des Tilleuls**
**F-92350 Le Plessis-Robinson (FR)**

(74) Mandataire : **Bernasconi, Jean et al**
**Cabinet Michel Lemoine 13, Bd. des Batignolles**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Tête de mesure permettant l'obtention de signaux de mesures physiologiques, destinée à être mise en place sur ou dans des parties corporelles

La présente invention concerne une tête de mesure combinée, destinée à être mise en place sur ou dans des parties corporelles, permettant l'obtention de signaux de mesures relatifs à une activité électrique d'une part, et à une activité ionique ou physico-chimique d'autre part.

On connaît d'après la réédition 28 990 du brevet des Etats-Unis d'Amérique 3.827.428, une tête de mesure disposée à l'intérieur de l'extrémité d'un tube flexible, cette tête portant une électrode axiale sensible à l'activité électrique et une contre-électrode disposée en spirale autour de ladite électrode et présentant une extrémité en forme de griffe. Pendant l'introduction, la tête est escamotée dans ce tube qui permet ensuite le coulissement et la rotation de la tête lors de la mise en place. Une tête de mesure ayant une forme voisine mais sans aucune gaine de protection est également décrite dans le brevet français 2 247 725.

Une tête présentant une double griffe constituée de deux spirales entrelacées est par ailleurs décrite dans le brevet français 2.120.787 avec possibilité d'adjonction d'une canule. Enfin, le brevet français 2.118.657 décrit une tête de mesure présentant également une double griffe en forme de deux spirales imbriquées l'une dans l'autre avec un tube extérieur de protection pour l'introduction dans une partie corporelle.

Ces configurations présentent un certain nombre d'inconvénients.

Ainsi la pose de ces têtes nécessite un mouvement de rotation dont l'amplitude ne peut pas être contrôlée facilement en l'absence de vision directe, de sorte que l'on peut être amené à trop visser les griffes en forme de spirale et causer des blessures ou au contraire à ne pas les visser assez avec en conséquence, le risque d'une séparation intempestive ultérieure de la tête d'avec l'organe sur lequel elle est fixée. En outre, même en cas de rotation exagérée, rien ne s'oppose dans la pratique pendant le fonctionnement, par exemple pendant un accouchement, à une rotation en sens inverse, tendant à séparer la tête de l'organe en question.

Pour celles de ces têtes qui portent une griffe spirale et une électrode pointue dirigée axialement, la pénétration axiale n'est pas optimale car elle diminue la précision du positionnement de l'électrode par rapport au tissu rencontré et d'autre part, risque de provoquer, en cas de vissage exagéré des griffes en forme de spirale, un accroissement de la plaie sans pour cela s'opposer en aucune manière à une séparation intempestive de la tête d'avec l'organe récepteur.

En outre, ces têtes déjà connues ne sont pas adaptées à l'obtention de signaux de mesure relatifs à la fois à une activité électrique et à une activité ionique ou physico-chimique telle que par exemple le pH.

Un autre inconvénient découle du fait que l'électrode de référence, associée à l'électrode de mesure de pH, est le plus couramment constituée d'un fil d'argent-chlorure d'argent plongeant dans une solution de KCl qui doit être maintenue dans un réservoir de capacité suffisante pour pouvoir humecter la partie corporelle en contact avec l'électrode de référence pendant toute la durée de la mesure. Ce réservoir, se trouvant situé dans le corps de la tête de mesure, présente en conséquence une capacité limitée, ainsi qu'un moyen d'accès, pour son remplissage éventuel au cours de la mesure, peu accessible.

Il a paru utile, en conséquence, de créer un nouveau dispositif, objet de la présente invention, permettant d'éliminer les inconvénients précités et, notamment, de mettre en place la tête de mesure en une seule opération, sans utilisation d'accessoire de pose, sans risques de blessures pendant sa pose sur la partie corporelle ; de créer une nouvelle structure de tête de mesure qui puisse, malgré des mouvements d'amplitude importante de la partie où la tête de mesure est appliquée, supporter des efforts importants, sans déformation ou cassure de tout ou partie de celle-ci ; créer une fixation de la tête de mesure qui, malgré ces mouvements, ne puisse se relâcher sans intervention humaine extérieure afin d'éviter, en conséquence, des discontinuités dans les signaux de mesure et une mauvaise interprétation de ceux-ci ; de réaliser une électrode de référence, associée à l'électrode de mesure de l'activité ionique tissulaire, dont le réservoir soit de capacité importante, et dont l'orifice de remplissage soit d'accès aisé, y compris au cours de la mesure.

Un des buts de l'invention est de constituer une tête de mesure, combinée, permettant l'obtention simultanée de signaux de mesures relatifs à une activité électrique ainsi qu'à une activité ionique, ou physico-chimique, de la partie corporelle où elle est appliquée, et dont la ppose se fasse en une seule opération, sans utilisation d'accessoire spécial de protection de la griffe lors de la pose et sans risque de déchirer la surface de la partie corporelle, sur ou dans laquelle elle est placée, pendant son introduction.

Un autre but de l'invention est de réaliser une tête de mesure qui, une fois mise en place sur ou dans la partie corporelle, soit mécaniquement stable, malgré les mouvements de la partie où la tête de mesure est appliquée, et qui ne puisse pas être placée en porte-à-faux, à la suite de ses mouvements, éliminant ainsi les risques de cassure ou de déformation des électrodes.

Un autre but de l'invention est de réaliser une tête de mesure dont le dispositif de fixation ne puisse se dégager, sans intervention humaine extérieure, par suite des mouvements de la partie sur laquelle elle est appliquée, afin d'éviter que les signaux de mesures soient interrompus.

Un autre but de l'invention est de réaliser une tête de mesure dont l'électrode, sensible à une activité ionique, fasse partie intégrante du dispositif de fixation mécanique de celle-ci, sur la partie corporelle où elle est appliquée, en association avec la ou les électrodes sensibles à l'activité électrique à mesurer.

Un autre but de l'invention est de réaliser une tête de mesure, dotée d'une électrode de référence pouvant être au KCl, dont le réservoir soit de grande capacité et soit doté d'un orifice de remplissage d'accès facile, de telle sorte qu'il puisse être alimenté de l'extérieur, éventuellement de manière continue sans que, pour autant, les dimensions externes de la tête de mesure soient augmentées et sans que la mesure soit interrompue.

Un autre but de l'invention est de réaliser une tête de mesure, dotée d'une électrode de référence pouvant être au KCl, qui soit susceptible d'être remplacée facilement.

L'invention a donc pour objet une tête de mesure permettant l'obtention de signaux de mesure physiologiques, notamment de signaux de mesure relatifs à une activité électrique et de signaux de mesure relatifs à une activité ionique ou physico-chimique, notamment le pH, destinée à être mise en place sur ou dans des parties corporelles, notamment sur la tête d'un enfant non encore né, ladite tête comprenant un corps isolant présentant au moins une première électrode en forme d'aiguille sensible à l'activité électrique et une électrode de référence associée à cette dernière ainsi que des moyens conducteurs, une gaine flexible reliée à une première extrémité audit corps isolant et contenant lesdits moyens conducteurs, et au moins une griffe d'accrochage pouvant faire fonction d'électrode sensible à l'activité électrique et escamotable lors de l'introduction de la tête de mesure dans une cavité corporelle ou lors de son extraction hors de ladite cavité, des moyens de réglage étant disposés à la seconde extrémité de la gaine pour commander l'escamotage de la ou des griffes, caractérisée en ce qu'elle comporte au moins une seconde électrode en forme d'aiguille sensible à une activité physique ou ionique et une électrode de référence, associée à cette dernière ainsi que des moyens conducteurs correspondants, en ce que ladite ou lesdites griffes d'accrochage sont de forme semi-circulaire et· montées de façon mobile sur le corps isolant et rétractable à l'intérieur de celui-ci et en ce que lesdites électrodes en forme d'aiguille(s) sont également montées de façon mobile sur le corps isolant et rétractable à l'intérieur de ce dernier, la direction de pénétration de ladite électrode sensible à une activité physique ou ionique étant opposée à celle de ladite ou desdites griffes.

De façon particulièrement avantageuse, ladite électrode sensible à l'activité ionique pénètre dans l'organe correspondant, par exemple le scalp de l'enfant non encore né, avec un très faible angle d'incidence par rapport à la surface dudit organe, la direction de pénétration étant de préférence opposée à la direction de pénétration des électrodes en forme de griffes mobiles et rétractables. De façon particulièrement préférée, lesdites griffes mobiles et rétractables et ladite électrode sensible à l'activité ionique sont liées mécaniquement pour être, simultanément, sorties ou rétractées.

De façon avantageuse, le boîtier isolant peut présenter, au niveau de la zone de pénétration de l'électrode sensible à l'activité ionique ou physique et/ou des griffes, des évidements provoquant le passage d'une discontinuité dans la courbure de la surface cutanée pour faciliter le perçage de la peau et la fixation.

Toujours dans cette forme de réalisation préférée, la tête de mesure selon l'invention peut avantageusement comporter une gaine flexible dont une des extrémités est mise en butée sur une extrémité correspondante du boîtier, l'autre extrémité de la gaine comportant un moyen de réglage, ladite gaine entourant un prolongement flexible du corps de ladite électrode sensible à l'activité ionique ou physique ainsi que celui de son électrode de référence associée, les conducteurs électriques des signaux de mesure et une pièce mécanique longiligne et flexible transmettant auxdites électrodes mobiles et rétractables le mouvement de translation obtenu par ledit moyen de réglage.

L'électrode de référence associée, qui peut être notamment une électrode au KCl, se prolonge avantageusement sur toute la longueur de la gaine pour former ainsi un réservoir agrandi de KCl. Cette électrode se termine avantageusement au niveau de la surface du boîtier appliqué contre la peau de l'organe correspondant, par un moyen de limitation poreux du KCl, par exemple une céramique poreuse ou une pastille de matériau convenable.

En variante, ledit boîtier peut être muni d'une ou deux électrodes de référence jetables telles que, par exemple, une plaque en KCl gélifiée protégée par un revêtement poreux, par exemple en papier. En variante également, la tête peut présenter un capot amovible jetable, ledit capot présentant un ou plusieurs réservoirs de KCl, ainsi qu'un ou plusieurs contacts électriques associés et susceptibles de s'appliquer, au moment du montage de l'embase, sur un contact complémentaire présenté par la partie réceptrice du boîtier, la transmission électrique s'effectuant par un conducteur convenable à partir de ce dernier contact.

Afin de faciliter éventuellement un remplacement des moyens d'accrochage, qui sont de préférence des griffes recourbées sollicitées par des moyens élastiques en position enfoncée et susceptibles d'être rappelées, à l'encontre desdits moyens élastiques, par le dispositif de réglage extérieur, on peut prévoir que le boîtier porte une ou plusieurs pinces métalliques susceptibles de recevoir l'extrémité fixe desdits moyens d'accrochage pour assurer la continuité électrique.

D'autres avantages et caractéristiques de l'invention apparaîtront à la lecture de la description suivante, faite à titre d'exemple non limitatif, et se référant au dessin annexé dans lequel :

la figure 1 donne le schéma de la tête de mesure selon la version préférée de l'invention ;

la figure 2 donne le détail d'un mécanisme simple permettant de lier le mouvement de l'électrode sensible à l'activité ionique au mouvement de rotation de la griffe ;

la figure 3 donne une vue schématique d'un moyen de réglage de la tête selon l'invention ;

la figure 4 donne une vue en coupe d'une tête selon une variante de l'invention ;

la figure 5 donne une vue de dessus de cette tête, le capot amovible et jetable étant représenté en traits mixtes ;

la figure 6 donne une vue analogue à la figure 4, mais avec les électrodes rétractées ;

les figures 7, 8 et 9 donnent des vues latérales, de dessus et de bout de l'embase de tête ;

les figures 10, 11 et 12 donnent des vues en coupe latérale, en demi-coupes de bout et de dessus, du capot amovible ;

la figure 13 donne une vue partielle du capot dans une variante à électrode de référence jetable ;

la figure 14 donne une vue latérale des moyens de réglage externes.

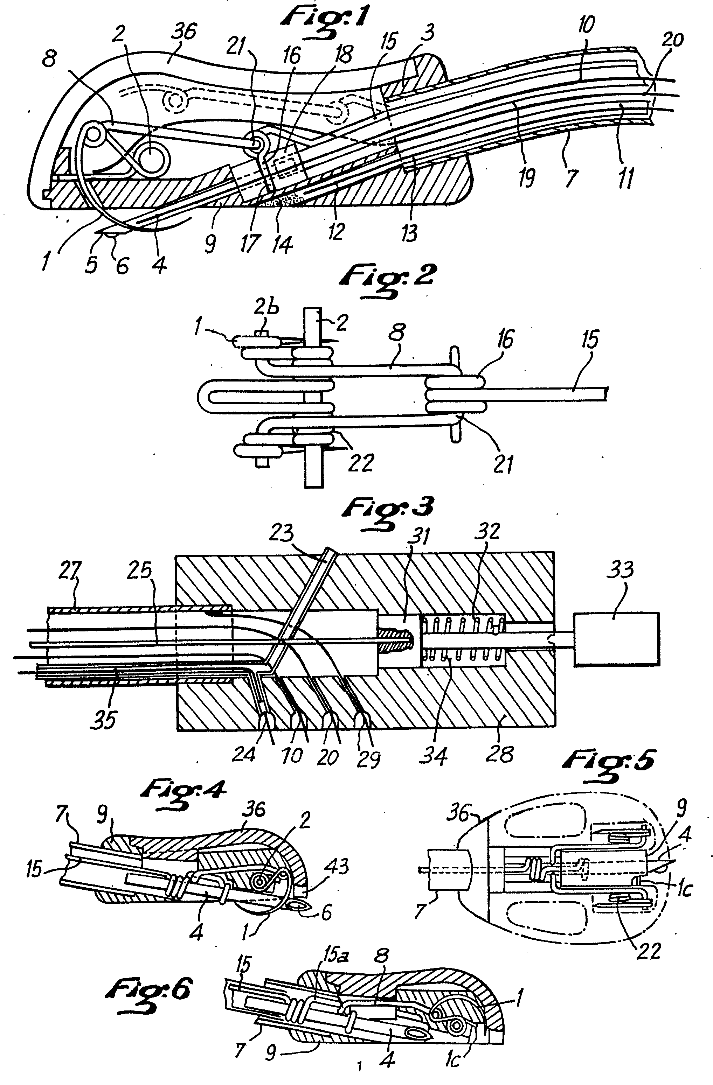

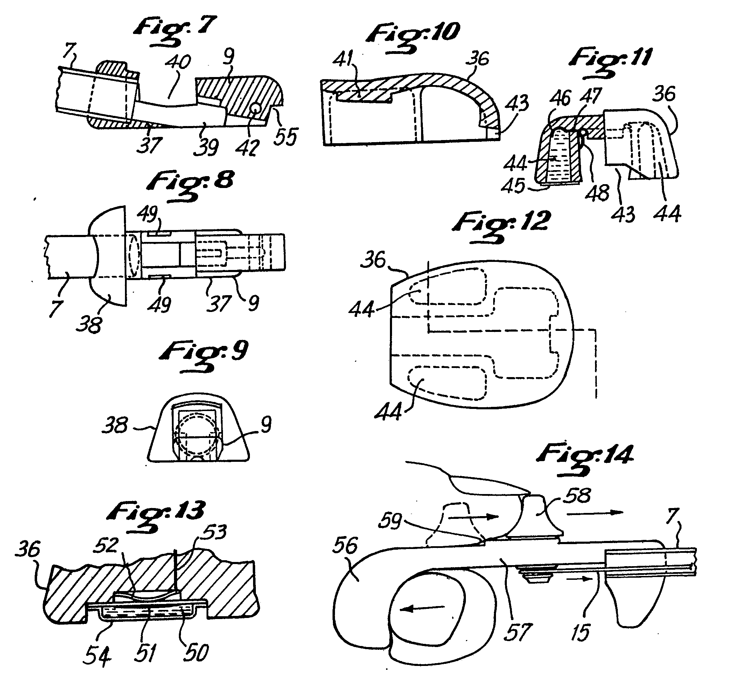

Suivant le mode de réalisation de la figure 1, la tête de mesure isolante comporte une griffe 1, simple ou double, de forme semi-circulaire, mobile autour d'un axe horizontal 2, parallèle à la surface corporelle où la tête de mesure est appliquée, pouvant sortir ou rentrer, hors ou à l'intérieur du boîtier 3 formant le corps de la tête de mesure, par un mouvement de rotation et permettant de recueillir, lorsqu'elle est enfoncée dans la partie corporelle, un signal relatif à l'activité électrique de cette dernière. La tête de mesure comporte d'autre part une électrode sélective 4, sensible à l'activité de l'ion hydrogène, comprenant un fin tube d'acier inoxydable 5 à l'extrémité biseautée, contenant et protégeant l'électrode sélective proprement dite et laissant légèrement dépasser, du côté de l'extrémité biseautée, une membrane sensible 6 pouvant être en verre. Cette électrode 4, de forme rectiligne, est dotée d'un mouvement de translation linéaire, selon un axe incliné coïncidant avec l'axe d'une gaine cylindrique flexible 7, venant elle-même en butée sur le boîtier 3 et se prolongeant entre les deux griffes la précédant, au niveau de la partie corporelle.

L'électrode sélective 4 est avantageusement une électrode de détection de pH d'un type usuel, vendu dans le commerce. On voit que la direction de pénétration de cette électrode est opposée à la direction de pénétration des griffes 1, ce qui assure une fixation solide de la tête de mesure.

Une pièce mécanique mobile 8, reliant l'électrode sélective 4 à la double griffe 1, permet de transformer le mouvement de translation de la première en un mouvement de rotation de la seconde. Cette opération se fait de telle sorte que, lorsque l'électrode sélective 4 est poussée vers le bas dans la partie corporelle selon un mouvement linéaire, la double griffe 1 s'enfonce également dans cette même partie corporelle selon un mouvement de rotation, de telle sorte que la griffe 1 s'oppose au dégagement de l'électrode sélective 4.

L'électrode sélective 4 est guidée dans son mouvement de translation, du côté de la partie corporelle, par une embase 9 de la tête de mesure et, à son autre extrémité, par la gaine flexible 7 pouvant être réalisée à l'aide d'un fil d'acier spiralé, à spires jointives. Cette gaine flexible 7, suffisamment rigide pour protéger les éléments qu'elle contient, entoure le câble électrique 10 de l'électrode sélective 4, entoure un prolongement 11 du réservoir d'une électrode de référence 12 au KCl, pouvant être constituée d'un fin tube de polyéthylène 13 se terminant par un matériau poreux 14, destiné à limiter la quantité de KCl s'écoulant de l'électrode de référence, et ayant une section suffisante pour que les particules ou corps d'origine biologique ne parviennent pas à l'obstruer. La position de cet orifice, ainsi que sa section, sont choisies de manière à assurer le meilleur contact électrique possible entre la partie corporelle et l'électrode de référence au KCl.

La gaine flexible 7 entoure également un conducteur électrique 20 connecté à la double griffe 1, ainsi qu'une pièce mécanique 15, pouvant être un fil d'acier suffisamment rigide et flexible, fixé à l'intérieur du boîtier 3 par l'intermédiaire d'un collier 16 pouvant être amovible et serré autour de l'électrode sélective 4, au niveau d'une gorge 17 pratiquée dans une bague isolante 18 dont le rôle est, par ailleurs, d'améliorer la qualité de la jonction réalisée entre la partie rigide 5 de l'électrode sélective 4 et un prolongement flexible pouvant être un tube de polyéthylène 19. Un axe de fixation mobile 21 maintient le collier de serrage 16, la pièce mécanique mobile 8 et la pièce mécanique mobile 15 assemblés.

La partie en pointillés, représentant la pièce mécanique mobile 8, correspond à une position où les deux types d'électrodes sont mis en position rétractée, à l'intérieur du boîtier. Dans cette position, ni la griffe semi-circulaire 1, ni l'électrode-aiguille 4 ne peuvent entrer en contact avec la partie corporelle.

La figure 2 donne le détail d'une réalisation possible d'un mécanisme de coordination des mouvements entre l'électrode en forme de griffe semi-circulaire 1 et l'électrode sélective 4, dotée d'un mouvement de translation linéaire. Il comprend une pièce mécanique mobile 8, reliée d'une part à un axe de fixation mobile 21, solidaire de l'électrode sélective 4 sensible à l'activité ionique, et d'autre part à un axe de fixation mobile 2, solidaire d'une double griffe 1. Un axe de fixation 2, fixe, est solidaire du corps de la tête de mesure.

Il peut être entouré de plusieurs spires 22, parties intégrantes de la double griffe 1, pouvant faire office de ressort de rappel, contribuant, soit à introduire les électrodes dans la partie corporelle, soit à les rétracter à l'intérieur du boîtier. Le mouvement de l'ensemble est commandé par la pièce 15, par l'intermédiaire d'un collier 16 et de l'axe mobile 21.

La figure 3 donne une présentation possible du moyen de réglage, placé à l'autre extrémité 27 d'une gaine flexible, repérée en 7 sur la figure 1. Ce moyen de réglage peut être constitué d'un corps 28, en matériau isolant, recevant également en butée l'autre extrémité 27 de la gaine 7. Il contient par ailleurs une cavité cylindrique 34 destinée à guider l'autre extrémité 25 de la tige flexible, dénommée 15 sur la figure 1. Celle-ci se prolonge, à l'extérieur, par une poignée 33 et comporte, dans le cylindre 34, un ressort de rappel 32 ainsi qu'une bague 31 qui en est solidaire. Ce ressort, butant contre la bague 31 et contre le corps du boîtier 28 permet, par la pression qu'il exerce sur la tige 15, de maintenir les deux électrodes, dénommées 1 et 4 sur la figure 1, en position enfoncée et, en conséquence, de maintenir la tête de mesure fixée sur la partie corporelle où elle est appliquée. Il est possible, sans sortir du cadre de l'invention, de concevoir un tout autre moyen de réglage de la position des électrodes.

Le boîtier 28 maintient par ailleurs deux conducteurs électriques 29 et 20 fournissant le signal relatif à l'activité électrique de la partie corporelle. Le conducteur 20 est relié à la double griffe semi-circulaire. Le conducteur 29 est présenté, ici, électriquement connecté à la gaine métallique 7. Il pourrait être réalisé d'une autre façon, l'essentiel étant qu'il soit relié à l'électrode de référence associée à et/ou aux griffes permettant d'obtenir le signal relatif à l'activité électrique de la partie corporelle. Le boîtier maintient également des conducteurs électriques 24 et 10. Le conducteur 10 est le conducteur interne de l'électrode sélective sensible. Le conducteur 24 est le conducteur interne de l'électrode de référence 12 au KCl associée à l'électrode sélective. Il est à noter que ce conducteur peut remplacer le conducteur 29 et servir, pour le signal relatif à l'activité électrique, de conducteur électrique de référence.

L'extrémité 35 du tube 13 se termine, à l'intérieur du boîtier 28, en une double dérivation, l'une contenant le conducteur électrique 24, l'autre aboutissant à l'orifice de remplissage 23. Cet orifice peut être le plus simplement constitué par le prolongement du tube en polyéthylène, constituant le prolongement du corps de cette même électrode de référence au KCl, ou par tout autre dispositif permettant le remplissage de celle-ci en KCl, en mode continu ou non, pendant la mesure ou non, sans que l'on sorte du cadre de l'invention.

Selon les descriptions données sur les figures 1, 2, 3, l'ensemble de la tête de mesure s'utilise en plaçant dans une main le boîtier contenant les électrodes de fixation, l'index étant placé sur celui-ci, dans un creux prévu à cet effet, de manière à diriger au « toucher » celle-ci à l'intérieur de la partie corporelle.

Le boîtier 28 est maintenu de l'autre main en prenant soin, lors de la pose, de maintenir tirée la poignée 33 de la figure 3 et, en conséquence, de maintenir rétractées, à l'intérieur de leur boîtier, les électrodes de fixation afin de ne pas blesser la partie corporelle pendant la pose. La position de la tête de mesure sur la partie corporelle ayant été choisie, il suffit, tout en la maintenant en place, de relâcher la poignée 33 liée au câble 25 qui, sous l'effet du ressort 32 qui était en position de compression poussant la bague 31 solidaire du cable 25, a pour effet de faire avancer l'électrode sélective 4 de la figure 1 dans la partie corporelle, ainsi que les griffes 1 représentées sur la même figure. Pour retirer l'électrode de la partie corporelle, il faut suivre l'opération inverse.

On se référera maintenant aux figures 4 à 12 sur lesquelles les éléments homologues sont désignés par les mêmes chiffres de référence. L'embase 9 de la tête reçoit, de façon amovible, un capot ou couvercle 36. La forme de l'embase est vue sur les figures 7 à 9. Elle comporte une partie rectiligne 37 avec une extrémité postérieure élargie 38 ayant sensiblement la largeur du capot 36 et l'embase est traversée par un passage 39 légèrement incliné par rapport à sa face inférieure, étant élargie dans la zone 38 pour permettre l'insertion et la fixation par blocage de l'extrémité de la gaine flexible 7. Devant la partie élargie 38, l'embase 9 présente un évidement supérieur 40 communiquant avec le passage 39 et dont les bords se rapprochent légèrement pour permettre la fixation par clippage du couvercle 36 présentant à cet effet un relief convenable 41.

L'embase 9 présente encore, vers sa partie antérieure, un orifice transversal 42 pour le passage de l'axe 2.

Le capot ou couvercle 36 présente la forme représentée sur les figures 10 à 12, cette forme sensiblement semi-ovoïde présentant, à l'extrémité antérieure, une échancrure 43. Lorsque l'on applique la tête du dispositif sur la peau d'un enfant non encore né, la peau a tendance à saillir tout autour de la tête et ce relief tend à pénétrer dans l'échancrure 43. Ceci défavorise ainsi la pénétration de l'extrémité de l'électrode-aiguille 4 dans la peau. On peut également, si on le désire, créer latéralement des échancrures pour faciliter la pénétration des griffes 1.

On peut avantageusement remplacer l'électrode de référence, en prévoyant dans le couvercle 36 deux orifices borgnes verticaux 44 débouchant à la face inférieure. On voit, sur les figures 11 et 12, la forme de ces orifices allongés parallèlement à l'axe de la tête. Ces orifices peuvent être obturés à leur face inférieure par un film poreux 45, par exemple en papier. L'orifice 44 est rempli d'une pâte de KCl ou autre, par exemple à base d'agar-agar. Le fond de l'orifice est tapissé par un film conducteur d'argent et de chlorure d'argent 46 relié par un conducteur 47 à un petit contact à lames 48 situé sur la face interne de la paroi interne de l'orifice 44. Le conducteur 47 traverse cette paroi. Les lames de contact 48 sont susceptibles de s'appliquer contre des lamelles métalliques correspondantes 49 logées dans des évidements latéraux de la partie 37 de l'embase 9 pour assurer le contact électrique. Les deux contacts 49 sont reliés au conducteur 24.

Les contacts 48, 49 peuvent être situés en tous autres endroits de l'embase ou du couvercle à l'abri du liquide amniotique.

En variante, on pourrait concevoir un couvercle non jetable analogue au couvercle 36, mais sans la présence des orifices 44. Il suffirait de prévoir, aux emplacements occupés par les deux films 45, des logements susceptibles de recevoir de façon amovible des pastilles formant électrodes de référence et contenant, de bas en haut, un film poreux 54 en papier, une couche de pâte 50 de chlorure de potassium, une pastille conductrice 51 d'argent/chlorure d'argent, ladite pastille d'argent venant au contact d'une pièce métallique 52 de contact présente dans le fond de l'évidement et connectée en 53. On voit une telle réalisation sur la figure 13.

La liaison entre l'aiguille-électrode 4 et l'élément flexible de commande 15, par exemple un fil d'acier, s'effectue de préférence comme on le voit sur les figures 4 à 6, en enroulant l'élément 15 autour de l'aiguille 4 de façon à former, entre deux enroulements successifs, un brin latéralement écarté 15a permettant l'accrochage de l'élément 8. Les griffes 1 elles-mêmes sont réalisées à partir d'un fil unique en acier à ressort dont les deux extrémités sont recourbées en forme de griffes 1, lesdites griffes se prolongeant par un certain nombre de spires 22 disposées autour de l'axe 2, lesdits groupes de spires étant reliés par un brin central 1c. On voit que ce brin central passe par un angle dièdre 55 situé à la partie antérieure de l'embase 9. Afin de permettre facilement le remplacement de la pièce constituée par les griffes 1, les enroulements faisant fonction de ·ressorts 22 et le brin central 1c, on peut prévoir, dans cet angle dièdre, une pince métallique (non représentée) dans laquelle le brin 1c vient se prendre de façon amovible pour être serré par cette pince. La pince est reliée au conducteur électrique 20 pour transmettre les signaux électriques détectés par les griffes.

En se référant à la figure 14, on voit un dispositif de commande prévu à l'extrémité extérieure de la gaine 7 et jouant un rôle analogue au dispositif représenté sur la figure 3. Ce dispositif représente une poignée de commande 56 contre laquelle vient se disposer l'index de l'opérateur. Cette poignée se prolonge par une partie rectiligne 57 munie d'une fente centrale dans laquelle est guidé de façon coulissante un doigt coulissant 58 susceptible d'être déplacé par le pouce. Sur ce doigt 58 se trouve fixée l'extrémité de l'élément flexible 15. On voit que la face supérieure du prolongement 57 présente un épaulement arrière 59 formant butée. Normalement, les griffes 1 se trouvent dans la position représentée sur la figure 6 et, dans ces conditions, la pièce 8 se trouve dans sa position arrière, l'électrode 4 n'émergeant pas de la base inférieure de la tête de mesure. Le doigt 58 se trouve alors dans une position arrière, position dans laquelle il se trouve maintenu en butée, dans la position représentée en traits interrompus, contre la butée 59. A partir de cette position l'opérateur, ayant introduit la tête de mesure dans le col de l'utérus et appliqué la base de ladite tête contre la peau cranienne de l'enfant non encore né, soulève la pièce 58 qui se trouve ainsi dégagée de la butée 59. Les spires 22 repoussent alors les griffes 1 en position active représentée sur la figure 4 et, dans ce mouvement, la pièce 8 transmet un effort de traction à l'élément 15 qui provoque l'enfoncement de l'électrode 4 dans sa position représentée sur la figure 4 et également le mouvement en avant du doigt 58. Le doigt a atteint alors la position avancée représentée sur la figure 14. Pour escamoter les griffes et l'électrode 4, l'utilisateur repousse le doigt 58 avec le pouce vers l'arrière pour le ramener dans la position représentée en traits interrompus.

Le cas échéant, si la force des spires 22 est insuffisante, l'utilisateur peut, en appuyant sur le doigt 58 pour le repousser vers l'avant, aider à la perforation de la paroi cutanée par les griffes 1.

Dans une variante, les griffes 1 peuvent ne pas servir d'électrodes et c'est alors le tube métallique 5 de l'électrode 4 qui, relié à un conducteur, par exemple la tige flexible 15, détecte les signaux électrocardiographiques. Dans tous les cas, on peut prévoir une contre-électrode ECG sur la gaine, venant en contact avec une partie corporelle maternelle.

**Revendications**

1. Tête de mesure permettant l'obtention de signaux de mesure physiologiques, notamment de signaux de mesure relatifs à une activité électrique et de signaux de mesure relatifs à une activité ionique ou physico-chimique, notamment le pH, destinée à être mise en place sur ou dans des parties corporelles, notamment sur la tête d'un enfant non encore né, ladite tête comprenant un corps isolant (3) présentant au moins une première électrode en forme d'aiguille sensible à l'activité électrique et une électrode de référence associée à cette dernière ainsi que des moyens conducteurs, une gaine flexible (7) reliée à une première extrémité audit corps isolant et contenant lesdits moyens conducteurs, et au moins une griffe d'accrochage (1) pouvant faire fonction d'électrode sensible à l'activité électrique et escamotable lors de l'introduction de la tête de mesure dans une cavité corporelle ou lors de son extraction hors de ladite cavité, des moyens de réglage (28) étant disposés à la seconde extrémité de la gaine (7) pour commander l'escamotage de 1a ou des griffes (1), caractérisée en ce qu'elle comporte au moins une seconde électrode en forme d'aiguille (4) sensible à une activité physique ou ionique et une électrode de référence (12, 44 + 45, 54 + 50), associée à cette dernière ainsi que des moyens conducteurs correspondants (10, 24), en ce que ladite ou lesdites griffes d'accrochage (1) sont de forme semi-circulaire et montées de façon mobile sur le corps isolant (3) et rétractable à l'intérieur de celui-ci et en ce que la ou lesdites électrodes en forme d'aiguille(s) sont également montées de façon mobile sur le corps isolant (3) et rétractable à l'intérieur de ce dernier, la direction de pénétration de ladite électrode sensible à une activité physique ou ionique étant opposée à celle de ladite ou desdites griffes (1).

2. Tête de mesure selon la revendication 1, caractérisée en ce que la ou lesdites griffes (1) constituent ladite première électrode sensible à l'activité électrique.

3. Tête de mesure selon la revendication 1, caractérisée en ce que lesdites première (5) et seconde (4) électrodes sont présentées par un même élément en forme d'aiguille.

4. Tête de mesure selon la revendication 3, caractérisée en ce que l'élément en forme d'aiguille comprend un tube métallique (5) faisant office d'électrode sensible à l'activité électrique.

5. Tête de mesure selon l'une des revendications 1 à 4, caractérisée en ce que les griffes (1), première électrode (1, 5) et seconde électrode (4) sont couplées mécaniquement pour être simultanément sorties hors du corps isolant (3) ou rétractées à l'intérieur de celui-ci.

6. Tête de mesure selon l'une des revendications 1 à 5, caractérisée en ce que ladite seconde électrode (4) se présente sous forme d'une aiguille rectiligne coulissante.

7. Tête de mesure selon la revendication 6, caractérisée en ce que lesdites griffes (1) et ladite aiguille coulissante sont reliées mécaniquement par une pièce (8) transmettant le mouvement de l'aiguille et vice versa.

8. Tête de mesure selon l'une des revendications 6 et 7, caractérisée en ce que lesdites griffes semi-circulaires (1) forment les deux extrémités d'un fil métallique comportant des spires (22) en forme de ressort sollicitant lesdites griffes (1) dans l'une des directions d'extraction et de rétraction dans le corps isolant (3).

9. Tête de mesure selon l'une des revendications 1 à 8, caractérisée en ce que le corps (3) présente une face hors de laquelle peuvent émerger les griffes (1), première électrode (1) et seconde électrode (4).

10. Tête de mesure selon la revendication 9, caractérisée en ce que ladite seconde électrode (4) émerge de ladite face du corps (3) selon une direction faiblement inclinée par rapport à ladite face.

11. Tête de mesure selon l'une des revendications 8 à 10, caractérisée en ce que ladite face est faiblement inclinée par rapport à la direction de la gaine (7).

12. Tête de mesure selon l'une des revendications 8 à 11, caractérisée en ce que ledit corps isolant (3) présente, en dehors de ladite face, une forme ovoïde.

13. Tête de mesure selon l'une des revendications 8 à 12, caractérisée en ce que ledit corps isolant (3) comporte un capot (36) monté de façon amovible sur une embase (9), ladite embase présentant ladite face par où émergent la ou les griffes (1), et les première et seconde électrodes (1, 5, 4).

14. Tête de mesure selon la revendication 13, caractérisée en ce que ladite embase (9) comporte une partie rectiligne (37) avec une extrémité postérieure élargie (38) ayant sensiblement la largeur du capot (36), l'embase étant traversée par un passage (39) légèrement incliné par rapport à sa face inférieure, ladite extrémité postérieure (38) étant reliée à la gaine (7) alors que devant elle l'embase (9) présente un évidement (40) communiquant avec le passage (39) dont les bords se rapprochent légèrement pour permettre la fixation par clipage du capot (36), un orifice transversal (42) étant présenté par la partie antérieure de l'embase pour le passage d'un axe de pivotement de griffe.

15. Tête de mesure selon l'une des revendications 8 à 14, caractérisée en ce qu'elle présente, au niveau de la zone de pénétration de ladite seconde électrode (4), un évidement (43) permettant le passage d'une discontinuité dans la courbure de la surface cutanée pour faciliter le perçage de la peau.

16. Tête de mesure selon l'une des revendications 8 à 15, caractérisée en ce que ladite face par où émergent l'aiguille (1) et les électrodes (1, 5, 4) présente une électrode de référence (14, 44 + 45, 50 + 54) associée à ladite seconde électrode (4) et notamment du type électrode au chlorure de potassium.

17. Tête de mesure selon la revendication 16, caractérisée en ce que ladite électrode de référence (14) se poursuit par un tube allongé (11) contenu dans la gaine (7) et aboutissant à un orifice de remplissage.

18. Tête de mesure selon la revendication 16 dans laquelle le corps comporte un capot (36) amovible, caractérisée en ce que ledit capot présente un ou plusieurs réservoirs de chlorure de potassium (44) obturés de façon à former une électrode de référence.

19. Tête de mesure selon l'une des revendications 1 à 18, caractérisée en ce que ladite gaine (7) contient une pièce flexible allongée (15) susceptible de coulisser à l'intérieur de la gaine (7) sous l'action de moyens de réglage (28, 33) et étant reliée au moins à ladite ou lesdites griffes (1) pour commander leur extraction ou rétraction par rapport au corps isolant (3).

20. Tête de mesure selon la revendication 19, caractérisée en ce que ladite pièce flexible allongée est électriquement conductrice et électriquement reliée à ladite première électrode (1, 5).

21. Tête de mesure selon l'une quelconque des revendications 1 à 20, caractérisée en ce que lesdits moyens de réglage présentent une forme de poignée (56, 57) avec un doigt mobile (58) de réglage susceptible d'être maintenu immobile contre une butée (59).

22. Tête de mesure selon l'une quelconque des revendications 1 à 21, caractérisée en ce que ladite électrode de référence associée à ladite première électrode (1, 5) est présentée par la surface extérieure de ladite gaine (7).

## Claims

1. A measuring head for obtaining physiological measurement signals, more particularly measurement signals relating to an electrical activity and measurement signals relating to an ionic or physico-chemical activity, more particularly the pH, intended for fitting on or in parts of the body, more particularly on the head of an unborn child, the measuring head comprising an insulating member (3) having at least one first electrode in the form of a needle sensitive to the electrical activity and a reference electrode associated with the latter and conductive means, a flexible sheath (7) connected at a first end to the said insulating member and containing the said conductive means, and at least one gripper claw (1) adapted to operate as an electrode sensitive to the electrical activity and retractable on introduction of the measuring head into a cavity of the body or on its removal therefrom, adjustment means (28) being disposed at the second end of the sheath (7) to control the retraction of the or each claw (1), characterised in that it comprises at least one second electrode in the form of a needle (4) sensitive to a physical or ionic activity and a reference electrode (12, 44 + 45, 54 + 50), associated with the latter and corresponding conductive means (10, 24), in that the said claw or claws (1) are of semi-circular shape and mounted movably on the insulating member (3) and retractable inside the latter and in that the or each electrode in the form of a needle is also mounted movably on the insulating member (3) and retractable inside the latter, the direction of penetration of the said electrode sensitive to a physical or ionic activity being the opposite to that of the said claw or claws (1).

2. A measuring head according to claim 1, characterised in that the or each claw (1) forms the said first electrode sensitive to the electrical activity.

3. A measuring head according to claim 1, characterised in that the said first electrode (5) and second electrode (4) are presented by a single needle-shaped element.

4. A measuring head according to claim 3, characterised in that the needle-shaped element comprises a metal tube (5) acting as the electrode sensitive to the electrical activity.

5. A measuring head according to any one of claims 1 to 4, characterised in that the claws (1), first electrode (1, 5) and second electrode (4) are mechanically coupled for simultaneous extension from the insulating member (3) or retraction inside the latter.

6. A measuring head according to any one of claims 1 to 5, characterised in that the second electrode (4) is in the form of a sliding rectilinear needle.

7. A measuring head according to claim 6, characterised in that the said claws (1) and the said sliding needle are mechanically connected by a member (8) which transmits the movement of the needle and vice-versa.

8. A measuring head according to claim 6 or 7, characterised in that the said semi-circular claws (1) form the two ends of a wire comprising turns (22) in the form of a spring urging the said claws (1) in one of the directions of extension from and retraction into the insulating member (3).

9. A measuring head according to any one of claims 1 to 8, characterised in that the member (3) has a surface from which the claws (1), the first electrode (1) and the second electrode (4) can emerge.

10. A measuring head according to claim 9, characterised in that the said second electrode (4) emerges from the said surface of the member (3) in a direction which is slightly inclined to said surface.

11. A measuring head according to any one of claims 8 to 10, characterised in that the said surface is slightly inclined to the direction of the sheath (7).

12. A measuring head according to any one of claims 8 to 11, characterised in that the said insulating member (3) is of ovoidal shape outside the said surface.

13. A measuring head according to any one of claims 8 to 12, characterised in that the said insulating member (3) has a cap (36) detachably mounted on a base (9), said base comprising the surface through which the or each claw (1), and the first and second electrodes (1, 5, 4) emerge.

14. A measuring head according to claim 13, characterised in that the said base (9) comprises a rectilinear part (37) with a widened rear end (38) of substantially the same width as the cap (36), there extending through the base a passage (39) which is slightly inclined to its bottom surface, the said rear end (38) being connected to the sheath (7) while in front of the sheath the base (9) has a recess (40) communicating with the passage (39), the edges of which are readily brought together to enable the cap (36) to be secured by snap engagement, a transverse aperture (42) being formed in the front part of the base for the passage of a claw pivot.

15. A measuring head according to any one of claims 8 to 14, characterised in that it is formed with a recess (43) at the level of the zone of penetration of the said second electrode (4), for the passage of a discontinuity in the curvature of the surface of the skin to facilitate the piercing thereof.

16. A measuring head according to any one of claims 8 to 15, characterised in that the said surface through which the needle (1) and the electrodes (1, 5, 4) emerge has a reference electrode (14, 44 + 45, 50 + 54) associated with the said second electrode (4), and more particularly of the potassium chloride electrode type.

17. A measuring head according to claim 16, characterised in that the said reference electrode (14) continues in the form of an elongate tube (11) contained in the sheath (7) and leading to a filling aperture.

18. A measuring head according to claim 16, in which the member has a detachable cap (36), characterised in that the said cap has one or more potassium chloride reservoirs (44) sealed off so as to form a reference electrode.

19. A measuring head according to any one of claims 1 to 18, characterised in that the said sheath (7) contains an elongate flexible part (15) adapted to slide inside the sheath (7) in response to the adjustment means (28, 33) and being connected at least to the or each claw (1) to control the extension or retraction thereof with respect to the insulating member (3).

20. A measuring head according to claim 19, characterised in that the said elongate flexible part is electrically conductive and electrically connected to the said first electrode (1, 5).

21. A measuring head according to any one of claims 1 to 20, characterised in that the said adjustment means are in the form of a handle (56, 57) with a movable adjustment finger (58) adapted to be held immovably against a stop (59).

22. A measuring head according to any one of claims 1 to 21, characterised in that the said reference electrode associated with the said first electrode (1, 5) comprises the outer surface of the said sheath (7).

**Ansprüche**

1. Meßkopf zur Darstellung physiologischer Meßsignale, insbesondere Meßsignale infolge elektrischer Aktivität und Meßsignale infolge ionischer oder physikochemischer Aktivität, insbesondere pH-Werte, der dazu bestimmt ist, an oder in Körperteilen, insbesondere am Kopf eines noch ungeborenen Kindes, eingesetzt zu werden ; besagter Meßkopf besteht aus einem Isolierkörper (3), der mindestens mit einer ersten, nadelförmigen, auf elektrische Aktivität empfindlichen Elektrode und mit einer Bezugselektrode, die mit der letzteren verbunden ist, sowie mit stromführenden Mitteln, mit einer flexiblen Hülle (7), die an einem Ende mit dem Isolierkörper verbunden ist und die die besagten stromführenden Mittel enthält, und mit mindestens einem Greifer (1), der als auf elektrische Aktivität empfindliche Elektrode dienen kann und der bei der Einführung des Meßkopfes in einen Körperhohlraum oder bei dessen Herausziehen aus diesem Hohlraum einziehbar ist, versehen ist, wobei Regulierungsmittel (28) an dem anderen Ende der Hülle (7) zur Steuerung des Einziehens des oder der Greifer (1) angebracht sind, dadurch gekennzeichnet, daß er mit mindestens einer nadelförmigen Elektrode (4), die auf physische und ionische Aktivität empfindlich ist, und mit einer mit der letzteren verbundenen Bezugselektrode (12, 44 + 45, 54 + 50) sowie mit entsprechenden stromführenden Mitteln (10, 24) versehen ist, daß der oder die Greifer (1) halbkreisförmig und auf dem Isolierkörper (3) beweglich angebracht und in diesen einziehbar sind und daß die nadelförmige(n) Elektrode(n) ebenfalls auf dem Isolierkörper (3) beweglich angebracht und in diesen einziehbar sind, wobei die Richtung des Eindringens der auf eine physische oder ionische Aktivität empfindliche Elektrode der Richtung der (oder des) Greifer(s) (1) entgegengesetzt ist.

2. Meßkopf nach Anspruch 1, dadurch gekennzeichnet, daß der oder die Greifer (1) die erste, auf elektrische Aktivität empfindliche Elektrode bilden.

3. Meßkopf nach Anspruch 1, dadurch gekennzeichnet, daß die erste Elektrode (5) und die zweite Elektrode (4) aus ein und demselben nadelförmigen Element gebildet sind.

4. Meßkopf nach Anspruch 3, dadurch gekennzeichnet, daß das nadelförmige Element aus einem Metallrohr (5) besteht, das als auf elektrische Aktivität empfindliche Elektrode dient.

5. Meßkopf nach einem der Ansprüche 1 bis 4, dadurch gekennzeichnet, daß die Greifer (1), die erste (1, 5) und die zweite (4) Elektrode mechanisch gekoppelt sind, um gleichzeitig aus dem Isolierkörper (3) ausgefahren oder in den Isolierkörper eingezogen werden zu können.

6. Meßkopf nach einem der Ansprüche 1 bis 5, dadurch gekennzeichnet, daß die zweite Elektrode (4) die Form einer geradlinigen verschiebbaren Nadel hat.

7. Meßkopf nach Anspruch 6, dadurch gekennzeichnet, daß die Greifer (1) und die verschiebbare Nadel durch ein Teil (8) mechanisch miteinander verbunden sind, das die Bewegung der Nadel überträgt und umgekehrt.

8. Meßkopf nach einem der Ansprüche 6 und 7, dadurch gekennzeichnet, daß die halbkreisförmigen Greifer (1) die beiden Enden eines Metalldrahtes bilden, welcher mit federförmig gedrehten Windungen (22) versehen ist, die die Greifer (1) in einer Ausfahr- bzw. Einziehrichtung in bzw. aus dem Isolierkörper (3) bewegen.

9. Meßkopf nach einem der Ansprüche 1 bis 8, dadurch gekennzeichnet, daß der Körper (3) eine Seite aufweist, aus welcher die Greifer (1), die erste (1) und die zweite (4) Elektrode austreten.

10. Meßkopf nach Anspruch 9, dadurch gekennzeichnet, daß die zweite Elektrode (4) aus der besagten Seite des Körpers (3) gemäß einer in Bezug auf diese Seite schwach geneigten Richtung austritt.

11. Meßkopf nach einem der Ansprüche 8 bis 10, dadurch gekennzeichnet, daß die besagte Seite in Bezug auf die Richtung der Hülle (7) schwach geneigt ist.

12. Meßkopf nach einem der Ansprüche 8 bis 11, dadurch gekennzeichnet, daß der Isolierkörper (3) außerhalb der besagten Seite eiförmig ist.

13. Meßkopf nach einem der Ansprüche 8 bis 12, dadurch gekennzeichnet, daß der Isolierkörper (3) mit einer Haube (36) versehen ist, die auf einem Sockel (9) abnehmbar aufgesetzt ist, wobei der Sockel die Seite bildet, aus welcher der oder die Greifer (1), die erste und die zweite Elektrode (1, 5, 4) austreten.

14. Meßkopf nach Anspruch 13, dadurch gekennzeichnet, daß der Sockel (9) ein geradliniges Teil (37) mit einem erweiterten hinteren Ende (38), das fühlbar dieselbe Breite hat wie die Haube (36), aufweist, wobei der Sockel von einem in Bezug auf seine Unterseite leicht geneigten Durchlaß (39) durchquert wird ; das erweiterte hintere Ende (38) mit der Hülle (7) verbunden ist, während vor diesem Ende der Sockel (9) eine Aussparung (40) aufweist, welche mit dem Durchlaß (39) in Verbindung steht, dessen Ränder sich leicht verjüngen, um die Befestigung der Haube (36) durch Klips zu ermöglichen und daß eine transversale Öffnung (42) für die Aufnahme einer Greiferschwenkachse im Vorderteil des Sockels vorhanden ist.

15. Meßkopf nach einem der Ansprüche 8 bis 14, dadurch gekennzeichnet, daß er in Höhe der Eindringzone der zweiten Elektrode (4) eine Aussparung (43) aufweist, welche die Aufnahme einer Diskontinuität in der Wölbung der Hautoberfläche ermöglicht, um das Durchstechen der Haut zu erleichtern.

16. Meßkopf nach einem der Ansprüche 8 bis 15, dadurch gekennzeichnet, daß die Seite, auf welcher die Nadel (1) und die Elektroden (1, 4, 5) austreten, eine mit der zweiten Elektrode (4) verbundene Bezugselektrode (11, 44 + 45, 50 + 54), insbesondere vom Typ einer Kaliumchlorid-Elektrode, aufweist.

17. Meßkopf nach Anspruch 16, dadurch gekennzeichnet, daß die Bezugselektrode (14) durch ein in der Hülle (7) enthaltenes längeres Rohr (11) weitergeführt wird, welches in eine Einfüllöffnung mündet.

18. Meßkopf nach Anspruch 16, bei welchem der Körper mit einer abnehmbaren Haube (36) versehen ist, dadurch gekennzeichnet, daß die Haube (36) einen oder mehrere Kaliumchlorid-Behälter (44) aufweist, die so abgedichtet sind, daß sie eine Bezugselektrode bilden.

19. Meßkopf nach einem der Ansprüche 1 bis 18, dadurch gekennzeichnet, daß die Hülle (7) ein längeres flexibles Teil (15) beinhaltet, das im Inneren der Hülle (7) mit Hilfe von Regulierungsmitteln (28, 33) verschoben werden kann, und das mit mindestens einem oder den beiden Greifern (1) verbunden ist, um deren Ausfahren bzw. Einziehen in Bezug auf den Isolierkörper (3) zu steuern.

20. Meßkopf nach Anspruch 19, dadurch gekennzeichnet, daß das längliche flexible Teil stromleitend ist und mit der ersten Elektrode (1, 5) elektrisch verbunden ist.

21. Meßkopf nach einem der Ansprüche 1 bis 20, dadurch gekennzeichnet, daß die Regulierungsmittel die Form eines Handgriffs (56, 57) mit einem beweglichen Schieberegler (58) aufweisen, der mit Hilfe eines Anschlags (59) festgehalten werden kann.

22. Meßkopf nach einem der Ansprüche 1 bis 21, dadurch gekennzeichnet, daß die mit der ersten Elektrode (1, 5) verbundene Bezugselektrode durch die Außenseite der Hülle (7) gebildet

0 004 510

Fig:1

Fig:2

Fig:3

Fig:4

Fig:5

Fig:6

Fig. 7

Fig. 10

Fig. 11

Fig. 8

Fig. 12

Fig. 9

Fig. 14

Fig. 13